# EUROPEAN PATENT SPECIFICATION

(11) **EP 2 060 993 B1**
(45) Date of publication and mention of the grant of the patent: **20.03.2013**
(21) Application number: 08168022.5
(22) Date of filing: 31.10.2008
(51) Int. Cl.: G08B 21/06, G06K 9/00, A61B 5/18

(54) **An awareness detection system and method**
Aufmerksamkeitserkennungssystem und Verfahren
Système et procédé de détection de sensibilisation

(30) Priority: 13.11.2007 US 983879
(43) Date of publication of application: 20.05.2009
(73) Proprietor: Delphi Technologies, Inc., Troy, MI 48007 (US)
(72) Inventor: Smith, Matthew R., Westfield, IN 46074 (US); Hammoud, Riad I., Kokomo, IN 46902 (US); Witt, Gerald J., Carmel, IN 46032 (US)
(74) Representative: Robert, Vincent

(56) References cited:
- EP-A- 0 990 416
- EP-A- 1 703 480
- JI Q ET AL: "Real-Time Nonintrusive Monitoring and Prediction of Driver Fatigue" IEEE TRANSACTIONS ON VEHICULAR TECHNOLOGY, IEEE SERVICE CENTER, PISCATAWAY, NJ, US, vol. 53, no. 4, 1 July 2004 (2004-07-01), pages 1052-1068, XP011115287 ISSN: 0018-9545
- SHAH M ET AL: "Determining driver visual attention with one camera" IEEE TRANSACTIONS ON INTELLIGENT TRANSPORTATION SYSTEMS, IEEE SERVICE CENTER, PISCATAWAY, NJ, US, vol. 4, no. 4, 1 December 2003 (2003-12-01), pages 205-218, XP011105769 ISSN: 1524-9050
- JI Q ET AL: "Real-Time Eye, Gaze, and Face Pose Tracking for Monitoring Driver Vigilance" REAL-TIME IMAGING, ACADEMIC PRESS LIMITED, GB, vol. 8, no. 5, 1 October 2002 (2002-10-01), pages 357-377, XP004419743 ISSN: 1077-2014

## Description

### Technical Field

The present invention generally relates to a system and method of awareness detection, and more particularly, to a system and method of detecting awareness based upon a subject's eye movement.

### Background of the Disclosure

Video imaging systems have been proposed for use in vehicles to monitor a subject person such as the driver and other passengers in the vehicle. Some proposed video imaging systems include one or two cameras focused on the driver of the vehicle to capture images of the driver's face. The video images are processed generally using computer vision and pattern recognition techniques to determine various facial characteristics of the driver including position, orientation, and movement of the driver's eyes, face, and head. Some advanced eye monitoring systems process the captured images to determine eye closure, such as open, half-open (half-closed), and closed states of the eye(s).

By knowing the driver's facial characteristics, vehicle control systems can provide enhanced vehicle functions. For example, a vehicle control system can monitor one or both eyes of the subject driver and determine a condition in which the driver appears to be fatigued or drowsy based on statistical analysis of the cumulated results of open or closed state of the eye(s) over time. Generally, standard human factor measures such as PerClos (percentage of eye closure) and AveClos (average of eye closure) could be used to determine the drowsiness state of the driver. For instance, if the AveClos value is determined to be above a certain threshold, the system may initiate countermeasure action(s) to alert the driver of the driver drowsy condition and/or attempt to awaken the driver.

Some proposed vision-based imaging systems that monitor the eye(s) of the driver of a vehicle require infrared (IR) illumination along with visible light filters to control scene brightness levels inside of the vehicle cockpit. One such driver monitoring system produces bright and dark eye conditions that are captured as video images, which are processed to determine whether the eye is in the open position or closed position. Such prior known driver eye monitoring systems generally require specific setup of infrared illuminators on and off the optical camera axis. In addition, these systems are generally expensive, their setup in a vehicle is not practical, and they may be ineffective when used in variable lighting conditions, especially in bright sunny conditions. Further, variations in eyelash contrast and eye iris darkness levels for different subject persons may cause such prior systems to make erroneous eye state discrimination decisions. EP-A-0 990 416 discloses a system which classifies a driver's eye direction as forward or downward.

### Summary of the Invention

A system and method in accordance with the present invention is defined by the features of claims 1 and 8 respectively.

According to one aspect of the present invention, an awareness detection system is provided. The system includes an imaging device positioned to obtain a plurality of images of at least a portion of a subject's head, and an awareness processor in communication with the imaging device. The awareness processor receives the plurality of images from the imaging device and performs the steps including classifying at least one image of the plurality of images based upon a head pose of at least a portion of the subject's head with respect to at least one image, monitoring movement of at least one eye of the subject if the at least one image is classified as a predetermined classification, and determining an awareness state of the subject based upon the monitored movement of the at least one eye, wherein the movement of at least one eye of the subject is monitored over at least two images obtained by the imaging device.

According to another aspect of the present invention, a method of detecting awareness of a subject is provided. The method includes the steps of obtaining a plurality of images of at least a portion of a subject, classifying at least one image of the plurality of images based upon a head pose of at least a portion of the subject's head with respect to at least one image, wherein the classification of the image includes one of frontal and non-frontal, monitoring movement of at least one eye of the subject if the at least one image is classified as a predetermined classification, and determining an awareness state of the subject based upon the monitored movement of at least one said eye, such that said awareness state includes one of distracted and non-distracted, wherein the movement of the at least one eye is monitored over at least two images.

These and other features, advantages and objects of the present invention will be further understood and appreciated by those skilled in the art by reference to the following specification, claims and appended drawings.

### Brief Description of the Drawings

The present invention will now be described, by way of example, with reference to the accompanying drawings, in which:
Fig. 1 is a top view of the front most portion of a passenger compartment of a vehicle equipped with an awareness detection system for monitoring a subject's driver, in accordance with one embodiment of the present invention;
Fig. 2 is a block diagram illustrating an awareness detection system, in accordance with one embodiment of the present invention;
Fig. 3 is a flow chart illustrating a method of detecting awareness of a subject in a vehicle, in accordance with one embodiment of the present invention;
Fig. 4 is a flow chart illustrating an exemplary method of detecting a head pose of a subject, in accordance with one embodiment of the present invention; and
Fig. 5 is a flow chart illustrating a method of monitoring movement of a subject's eyes, in accordance with one embodiment of the present invention.

### Description of the Preferred Embodiments

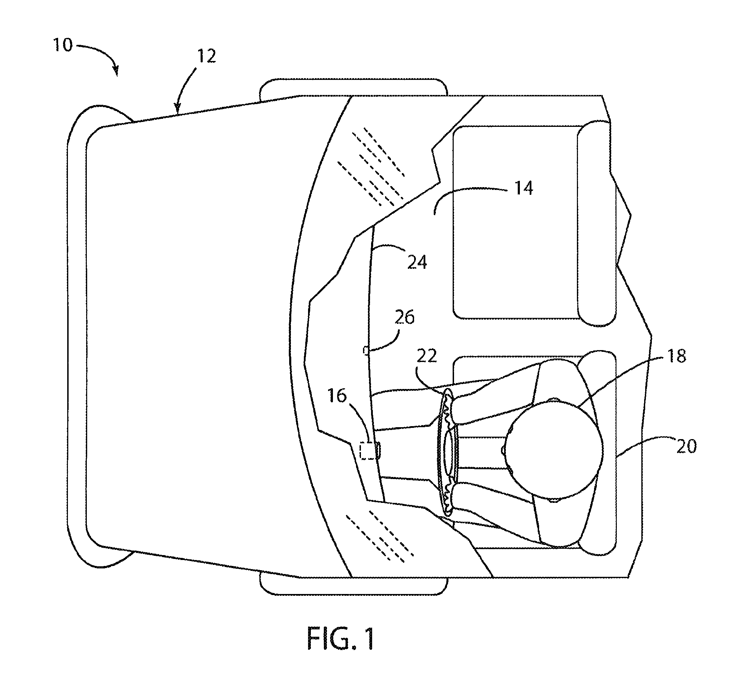

Referring to Fig. 1, an awareness detection system is generally shown at reference identifier 10. According to a disclosed embodiment, the awareness detection system 10 is used with a vehicle generally indicated at 12, such that the awareness detection system 10 is located inside a passenger compartment 14. The awareness detection system 10 has an imaging device 16 that obtains a plurality of images of at least a portion of a subject's 18 head. Thus, the awareness detection system 10 monitors the subject 18 to determine the awareness of the subject 18, as described in greater detail herein.

The imaging device 16 is shown located generally in front of a driver's seat 20 in the front region of the passenger compartment 14. According to one embodiment, the imaging device 16 is a non-intrusive system that is mounted in the instrument cluster. However, the imaging device 16 may be mounted in other suitable locations onboard the vehicle 12, which allow for acquisition of images capturing the subject's 18 head. By way of explanation and not limitation, the imaging device 16 may be mounted in a steering assembly 22 or mounted in a dashboard 24. While a single imaging device 16 is shown and described herein, it should be appreciated by those skilled in the art that two or more imaging devices may be employed in the awareness detection system 10.

The imaging device 16 can be arranged so as to capture successive video image frames of the region where the subject 18, which in a disclosed embodiment is typically driving the vehicle 12, is expected to be located during normal vehicle driving. More particularly, the acquired images capture at least a portion of the subject's 18 face, which can include one or both eyes. The acquired images are then processed to determine characteristics of the subject's 18 head, and to determine the awareness of the subject 18. For purposes of explanation and not limitation, the detected awareness of the subject 18 can be used to control other components of the vehicle 12, such as, but not limited to, deactivating a cruise control system, activating an audio alarm, the like, or a combination thereof.

According to one embodiment, the awareness detection system 10 can include a light illuminator 26 located forward of the subject 18, such as in the dashboard 24, for illuminating the face of the subject 18. The light illuminator 26 may include one or more infrared (IR) light emitting diodes (LEDs). Either on-access or off-access LEDs may be employed (e.g., no specific IR setup is required, in particular). The light illuminator 26 may be located anywhere onboard the vehicle 12 sufficient to supply any necessary light illumination to enable the imaging device 16 to acquire images of the subject's 18 head.

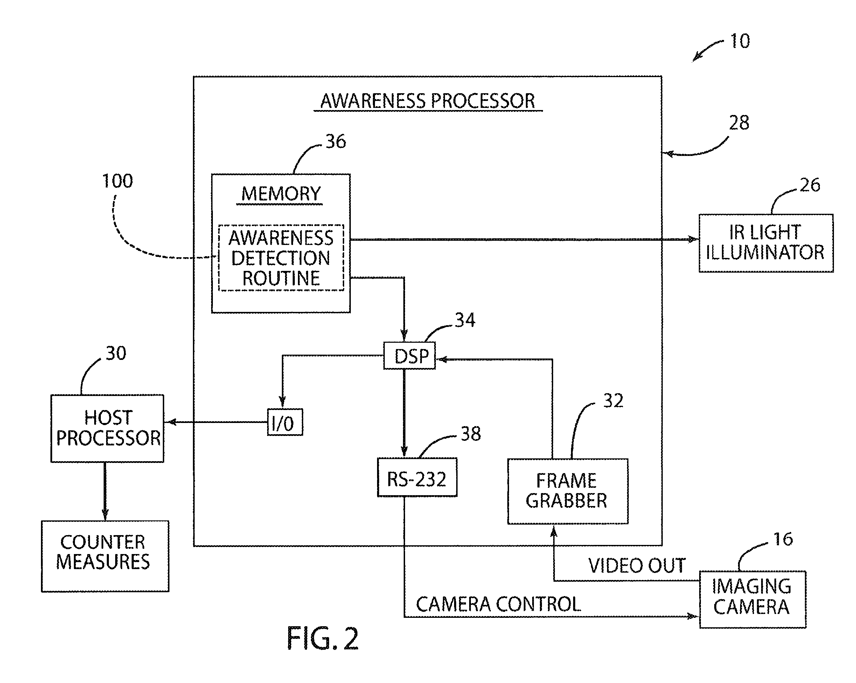

With regards to Fig. 2, the awareness detection system 10 is shown having the imaging device 16 and the light illuminator 26 in communication with an awareness processor generally indicated at 28. According to one embodiment, the light illuminator is an IR light source that emits light having a wavelength of approximately 940 nanometers (nm). Typically, the awareness processor 28 is in communication with a host processor 30. By way of explanation and not limitation, the imaging device 16 can be a CCD/CMOS active-pixel digital image sensor mounted as a chip onto a circuit board.

The awareness processor 28 can include a frame grabber 32 for receiving the video output frames generated by the imaging device 16. The awareness processor 28 can also include a digital signal processor (DSP) 34 for processing the acquired images. The DSP 34 may be a floating point or fixed point processor. Additionally, the awareness processor 28 can include memory 36, such as random access memory (RAM), read-only memory (ROM), and other suitable memory devices, as should be readily apparent to those skilled in the art. The awareness processor 28 is configured to perform one or more awareness detection routines for controlling activation of the light illuminator 26, controlling the imaging device 16, processing the acquired images to determine the awareness of the subject 18, and applying the processed information to vehicle control systems, such as the host processor 30.

The awareness processor 28 may provide imager control functions using a control RS-232 logic 38, which allows for control of the imaging device 16 via camera control signals. Control of the imaging device 16 may include automatic adjustment of the orientation of the imaging device 16. For purposes of explanation and not limitation, the imaging device 16 may be repositioned to focus on an identifiable feature, and may scan a region in search of an identifiable feature, including the subject's 18 head, and more particularly, one of the eyes of the subject 18. Also, the imager control may include adjustment of the focus and magnification as may be necessary to track identifiable features of the subject 18.

According to one embodiment, the awareness processor 28 is in communication with the imaging device 16, such that the awareness processor 28 receives the plurality of images from the imaging device 16, and performs the step of classifying at least one image of the plurality of images based upon at least a portion of the subject's 18 head. Additionally, the awareness processor 28 performs the steps of monitoring movement of at least one eye of the subject 18 if the at least one image is classified as a predetermined classification, and determining an awareness state of the subject 18 based upon the monitored movement of the at least one eye, as described in greater detail herein.

According to one embodiment, the subject 18 in at least one of the images is classified as frontal or non-frontal. For purposes of explanation and not limitation, the subject 18 is classified as frontal. If it is determined that the subject's 18 head is between approximately plus/minus twenty degrees (20°) from a straight-forward position, and the image is classified as non-frontal if the subject's 18 head is determined to be outside the plus/minus twenty degrees (20°) range. According to a disclosed embodiment, the predetermined classification for monitoring the movement of at least one eye of the subject 18 is a frontal classification, such that the eyes of the subject 18 are monitored only if the image is classified with a frontal classification.

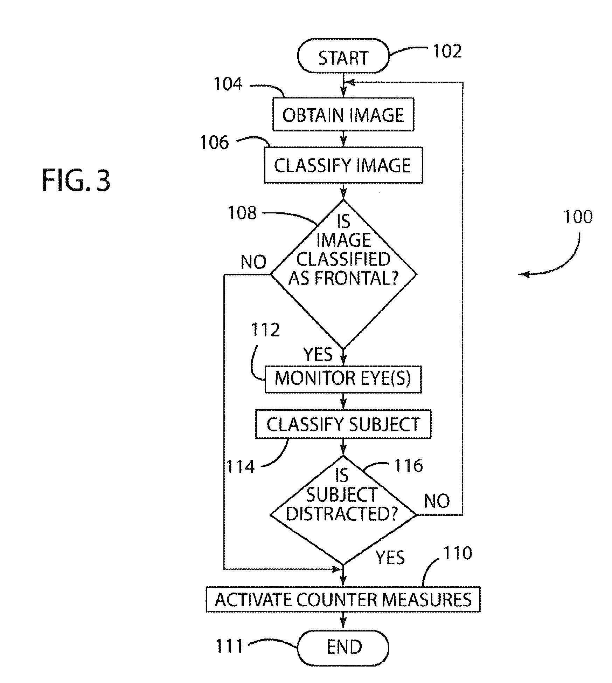

In reference to Figs. 1-3, a method of detecting awareness is generally shown in Fig. 3 at reference identifier 100. The method 100 starts at step 102, and proceeds to step 104, wherein an image is obtained. According to one embodiment, the imaging device 16 obtains at least one image of a subject 18 in a vehicle 12. At step 106, the image is classified. According to one embodiment, the awareness processor 28 obtains the image from the imaging device 16, and processes the image to classify the image as frontal or non-frontal. At decision step 108, it is determined if the image has been classified as frontal. If it is determined at decision step 108 that the image is not classified as frontal, then the method 100 can proceed to step 110, wherein counter measures are activated, since the subject can be classified as distracted, according to one embodiment. According to an alternate embodiment, if it is determined at decision step 108 that the image is not classified as frontal, then the subject 18 can be classified as distracted and the method can then end at step 111.

However, if it is determined at decision step 108 that the image is classified as frontal, the method 100 proceeds to step 112, wherein at least one eye of the subject 18 is located and is monitored over a plurality of images. Thus, the movement of the eyes can be monitored over a period of time to determine a pattern of eye movement. At step 114, the subject is classified based upon the detected eye movement at step 112. At decision step 116, it is determined if the subject 18 is distracted based upon the classification of the subject's 18 eye movements. If it is determined at decision step 116 that the subject 18 is distracted, then the method 100 can proceed to step 110, wherein counter measures are activated, according to one embodiment. According to an alternate embodiment, if it is determined at decision step 116 that the subject 18 is distracted, the method can then end at step 111. However, if it is determined that the subject 18 is not distracted at decision step 116, then the method 100 can return to step 104 to obtain an image. It should be appreciated by those skilled in the art that if it is determined at decision step 116 that the subject 18 is distracted, prior to or after counter measures are activated at step 110, the method 100 can return to step 104 to obtain an image.

According to one embodiment, the image classification at step 106 can include a head pose estimation, such as, but not limited to, an appearance based head pose estimation or a geometric based head pose estimation. According to a disclosed embodiment, predetermined facial features of the subject 18 can be extracted, such as, but not limited to, eyes, nose, the like, or a combination thereof. Thus, a face box or portion of the image to be analyzed or monitored can be determined based upon the extracted features. According to one embodiment, facial features of the subject 18 can be extracted from the image and compared to a three-dimensional (3D) head pose model. Alternatively, a head pose estimation can include detecting a face of the subject 18 and classifying the detected face online using a classification rule (i.e., distance) that can employ head pose appearance models built or constructed off-line. The head pose appearance models can be built during a testing phase, a development phase, or an experimental phase, according to one embodiment.

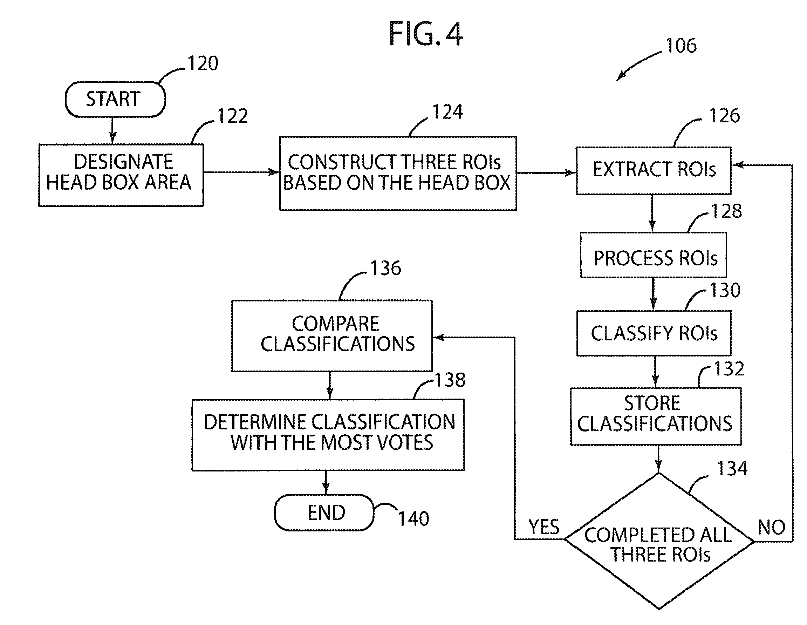

In reference to Figs. 1-4, an exemplary classification analysis of the image to determine a head pose of the subject 18 is generally shown in Fig. 4 at reference identifier 106, according to one embodiment. The classification analysis 106 starts at step 120, and proceeds to step 122, wherein at least a portion of the image received by the awareness processor 28 from the imaging device 16 is designated as the head box area. According to one embodiment, at step 124, three regions of interests (ROIs) are constructed based upon the head box from the image. According to a disclosed embodiment, a first ROI is the same size as the head box area defined at step 122, a second ROI is two-thirds (2/3) the size of the head box area defined in step 122, and a third ROI is four-thirds (4/3) the size of the head box area defined in step 122. For purposes of explanation and not limitation, the ROI sizes are twenty-four by twenty-eight (24x28) pixels, sixteen by eighteen (16x18) pixels, and thirty-two by thirty-six (32x36) pixels, respectively, according to one embodiment. Typically, the multiple ROIs that vary in size can be analyzed in order to reduce the effect of noise in the classification results. By way of explanation and not limitation, when the light emitter 26 emits IR light, the vehicle occupant's face and hair can reflect the IR while other background portions of the image absorb the IR. Thus, the head image may be noisy because of the IR being reflected by the hair and not just the skin of the vehicle occupant's face. Therefore, multiple ROIs having different sizes are used to reduce the amount of hair in the image in order to reduce the noise, which generally result in more accurate classifications.

At step 126, the three ROIs defined in step 124 are extracted from the image, and resized to a predetermined size, such that all three head boxes are the same size. At step 128, the awareness processor 28 processes the ROIs. According to a disclosed embodiment, the awareness processor 28 processes the ROIs by applying affine transform and histogram equalization processing to the image. It should be appreciated by those skilled in the art that other suitable image processing techniques can be used additionally or alternatively.

At step 130, each of the ROIs are designated or classified, wherein, according to one embodiment, the ROIs are given two classifications for two models, such that a first model is a normal pose model, and a second model is an outlier model. At step 132, the classifications results for the two models are stored. Typically, the classifications given to each of the head boxes for both the first and second classifications are left, front, or right.

At decision step 134, it is determined if the awareness processor 28 has processed or completed all the ROIs, such that the three ROIs have been classified and the results of the classification have been stored. If it is determined at decision step 134 that all three ROIs have not been completed, then the analysis 106 returns to step 126. However, if it is determined at decision step 134 that the awareness processor 28 has completed the three ROIs, then the analysis 106 proceeds to step 136. At step 136, the classifications are compared. According to a disclosed embodiment, the three ROIs each have two classifications, which are either left, front, or right, and thus, the number of front, left, and right votes can be determined. By way of explanation and not limitation, each ROI is classified as left, right, or front for both the normal pose model and the outlier model, and thus, there are a total of six classifications for the three ROIs, according to this embodiment. According to an alternate embodiment, each captured image has eighteen classifications, such that three ROIs at three different scales are constructed, wherein each ROI has three models and each model has two classifications. At step 138, the classification with the most votes is used to classify the image, and the classification analysis 106 then ends at step 140.

For purposes of explanation and not limitation, the outlier model can include a frontal image of the subject 18, such that the frontal classification is determined by patterns in the image that are not the subject's 18 eyes. The patterns can be, but are not limited to, the subject's 18 head, face, and neck outline with respect to the headrest. Thus, a head pose classification or analysis can be performed using such patterns or the like.

According to one embodiment, the awareness processor 28 determines the awareness state of the subject 18 as being one of distracted or non-distracted. According to a disclosed embodiment, the eyes of the subject 18 are monitored and plotted in a Cartesian coordinate plane in order to monitor the movement of the eye. Thus, a pattern of eye movement can be detected, such that a subject 18 can be determined to be distracted. According to one embodiment, at least one of the center of the subject's 18 eye is detected and tracked on an X-axis and Y-axis of a Cartesian coordinate plane, the movement of the subject's 18 eyelid (i.e., tightening or widening), an iris or pupil of the subject's 18 eye, or a combination thereof. Thus, the subject's 18 eye can be tracked as a whole, even though only a portion of the eye is detected or tracked. It should be appreciated by those skilled in the art that the detected eye movement can be tracked to classify the subject 18, even though the subject 18 is in a frontal position.

Exemplary systems and methods of monitoring the eye movement are United States Patent Application Serial No. 11/452,871 (US-A-2007/291983), entitled "METHOD OF TRACKING A HUMAN EYE IN A VIDEO IMAGE," , and United States Patent Application Serial No. 11/452,116 (US-A2007/286457), entitled "IMPROVED DYNAMIC EYE TRACKING SYSTEM," . An exemplary system and method of classifying an image of a subject as frontal and non-frontal is United States Patent Application Serial No. 11/890,066 (US-A-2009/034801), entitled "SYSTEM AND METHOD OF AWARENESS DETECTION," .

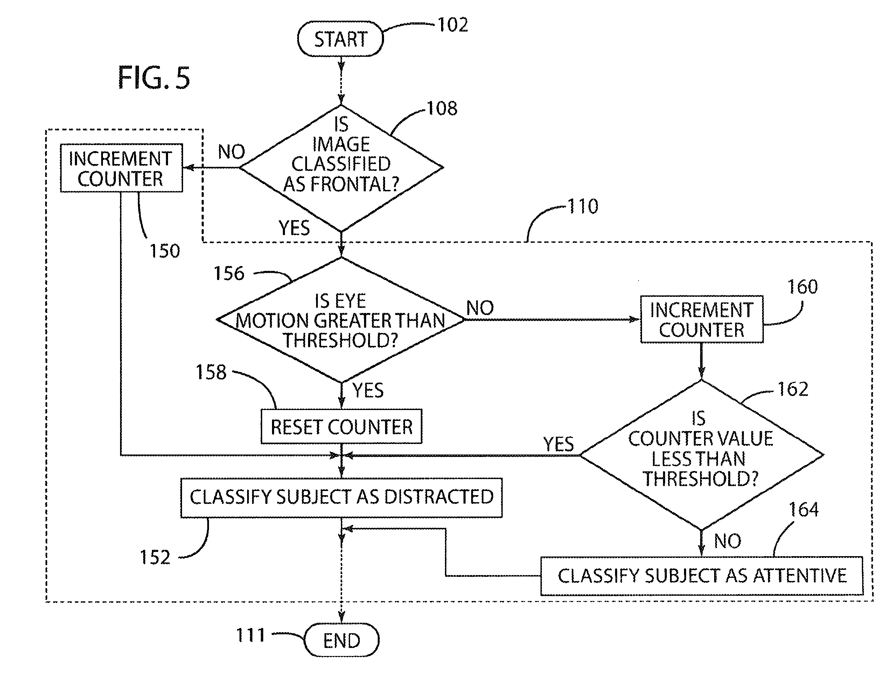

With regards to Figs. 1-5, a portion of method 100 is generally shown in Fig. 5, wherein the steps of determining if an image is classified as frontal (step 108) and monitoring the eye(s) (step 112) are generally shown, in accordance with one embodiment. At decision step 108, if it is determined that the image is not classified as frontal, then the eye motion analysis 112 is implemented, wherein a counter is incremented at step 150. The eye motion analysis 112 then proceeds to step 152, wherein the subject 18 is classified as distracted. The eye motion analysis 112 can then end, such that the method 100 can continue, as set forth above. However, if it is determined at decision step 108 that the image is classified as frontal, then the eye motion analysis 112 is implemented, wherein it is determined if the eye motion is above a first threshold value T₁ at decision step 156. According to one embodiment, the eyes of the subject 18 are monitored, wherein the motion of the subject's 18 eyes is given a representative numerical value that is compared to the threshold value T₁.

If it is determined at decision step 156 that the eye motion of the subject 18 is greater than the threshold value T₁, then the motion analysis 112 proceeds to step 158, wherein the counter is reset. According to one embodiment, when the counter is reset, the counter value equals zero. The motion analysis 112 then proceeds to step 152, wherein the subject 18 is classified as distracted, and the motion analysis 112 then ends, and the method 100 can continue, as set forth above.

However, it if is determined at decision step 156 that the eye motion is not above the threshold value T₁, then the motion analysis 112 proceeds to step 160, wherein the counter is incremented. According to a disclosed embodiment, the counter is incremented by a value of one (1). At decision step 162, it is determined if the value of the counter is less than a second threshold value T₂. If it is determined at decision step 162 that the counter value is less than the second threshold value T₂, then the motion analysis 112 proceeds to step 152, wherein the subject 18 is classified as distracted, and the motion analysis 112 then ends, and the method 100 can continue, as set forth above. If it is determined at decision step 162 that the counter value is greater than the second threshold value T₂, then the motion analysis 112 proceeds to step 164, wherein the subject 18 is classified as attentive. The motion analysis 112 then ends, and the method 100 can continue, as set forth above.

According to one embodiment, the value of the counter is incremented in order to prevent transient noise, such as, but not limited to, slight eye movement from affecting the classification of the subject 18. Thus, the value of the counter increases the longer the eye of the subject 18 has remained still and/or in substantially the same position. According to a disclosed embodiment, a high value in the counter represents that the eye of the subject 18 has remained substantially still, and thus, the subject 18 is attentive. By contrast, a low value in the counter can represent that the subject's 18 eye is moving, and thus, the subject 18 is distracted, according to one embodiment. Therefore, the threshold values T₁,T₂ can be predetermined accordingly, according to one embodiment.

According to one embodiment, the monitored eye motion is based upon the length of an X and Y motion vector. Typically, the motion vector is measured in pixels, according to a disclosed embodiment. According to one embodiment, the threshold value T₁ is predetermined, but is dependent upon how zoomed in the image is of the subject's 18 head. The threshold value T₂ represents 0.5 seconds, such that if the rate of conducting motion analysis 112 is ten times per second, the second threshold value T₂ would be five (5), according to one embodiment.

By way of explanation and not limitation, in operation, the awareness detection system 10 and method 100 determine the awareness of a subject 18, who can be a driver of a vehicle 12. The obtained image is first classified as frontal or non-frontal, and thus, if a non-frontal classification is designated, then it can be determined that the subject 18 is distracted. However, if the image is classified as frontal, then the eye movement of the subject 18 is monitored in order to determine if the subject 18 is distracted or non-distracted, since the subject 18 can have a frontal head position while being distracted based upon eye movement without head movement.

Advantageously, the awareness detection system 10 and method 100 accurately determine the awareness of a subject 18 based upon a fusion logic, such that the subject's 18 head positioning and eye movement. By additionally monitoring the eye movement of the subject 18 under predetermined circumstances, the awareness state of the subject 18 can more accurately be determined than if the determination was made solely on the head positioning of the subject 18. It should be appreciated by those skilled in the art that other motion detection techniques and eye detection or tracking techniques can be used in the fusion logic.

## Claims

1. An awareness detection system (10) comprising:
an imaging device (16) positioned to obtain a plurality of images of at least a portion of a subject's (18) head; and
an awareness processor (28) in communication with said imaging device (16), wherein said awareness processor (28) receives said plurality of images from said imaging device (16) and performs the steps comprising:
classifying at least one image (106) of said plurality of images based upon a head pose of at least a portion of said subject's (18) head with respect to at least one image of said plurality of images;
monitoring movement of at least one eye (112) of said subject if said at least one image is classified as a predetermined classification, wherein said monitoring includes characterizing an eye location as an X-Y co-ordinate on a Cartesian co-ordinate plane; and
determining an awareness state (114) of said subject (18) based upon said monitored movement of at least one said eye, wherein said movement of said at least one eye is monitored over at least two images of said plurality of images obtained by said imagine device (16), wherein said movement is indicated by a motion vector on the Cartesian co-ordinate plane;
wherein said classification of said image comprises one of frontal and non-frontal; and
wherein said predetermined classification is said frontal classification, such that at least one of said eyes is monitored after said subject (18) in at least one said image is classified as said frontal classification.

2. The awareness detection system (10) of claim 1, wherein said head pose classification comprises extracting at least one facial feature of said subject (18) from said image, and comparing said at least one extracted facial feature to a head pose model.

3. The awareness detection system (10) of claim 1, wherein said head pose classification comprises detecting at least a portion of a face of said subject (18), and classifying said detected face by a predetermined classification rule.

4. The awareness detection system (10) of claim 1, wherein said determined awareness state is one of distracted and non-distracted.

5. The awareness detection system (10) of claim 1, wherein said monitoring movement of at least one eye comprises comparing a length of the motion vector to a first threshold value (156).

6. The awareness detection system (10) of claim 5, wherein said monitoring movement of at least one eye further comprises comparing a value of a counter to a second threshold value (162).

7. The awareness detection system (10) of claim 1, wherein said awareness detection system (10) is used with a vehicle (12) to detect said subject (18) in said vehicle (12).

8. A method (100) of detecting awareness of a subject (18), said method (100) comprising the steps of:
obtaining a plurality of images (104) of at least a portion of a subject (18);
classifying at least one image (106) of said plurality of images based upon a head pose of at least a portion of said subject's (18) head with respect to at least one image of said plurality of images, wherein said classification of said at least one image comprises one of frontal and non-frontal;
monitoring movement of at least one eye (112) of said subject (18) if said at least one image is classified as a predetermined classification, wherein said monitoring includes characterizing an eye location as an X-Y co-ordinate on a Cartesian co-ordinate plane; and
determining an awareness state (114) of said subject (18) based upon said monitored movement of at least one said eye, such that said awareness state comprises one of distracted and non-distracted, wherein said movement of said at least one eye is monitored over at least two images of said plurality of images, wherein said movement is indicated by a motion vector on the Cartesian co-ordinate plane;
wherein said predetermined classification for said monitoring movement step to be performed is said frontal classification, such that at least one of said eyes is monitored after said subject (18) in at least one said image is classified as said frontal classification.

9. The method (100) of claim 8, wherein said head pose classification comprises extracting at least one facial feature of said subject (18) from said image, and comparing said at least one extracted facial feature to a head pose model.

10. The method (100) of claim 8, wherein said head pose classification comprises detecting at least a portion of a face of said subject (18), and classifying said detected face by a predetermined classification rule.

11. The method (100) of claim 8, wherein said step of monitoring movement of at least one eye comprises comparing a length of the motion vector to a first threshold value (156), such that it is determined that said subject (18) is in a first awareness state (152) when said counter value is greater than said first threshold value.

12. The method (100) of claim 11, wherein said step of monitoring movement of at least one eye further comprises comparing a value of a counter to a second threshold value (162), such that it is determined that said subject (18) is in said first awareness state (152) when said counter value is less than said second threshold value, and that said subject (18) is in a second awareness state (164) when said counter value is greater than said second threshold value.

13. The method (100) of claim 8, wherein said method (100) is used with a vehicle (12) to detect an awareness of said subject (18) in said vehicle (12).

## Patentansprüche

1. Ein Aufmerksamkeit-Erfassungssystem (10), das aufweist:
eine Abbildungsvorrichtung (16), die positioniert ist, um eine Vielzahl von Bildern von zumindest einem Teil eines Kopfes einer Person (18) zu erlangen; und
ein Aufmerksamkeit-Prozessor (28) in Kommunikation mit der Abbildungsvorrichtung (16), wobei der Aufmerksamkeit-Prozessor (28) die Vielzahl von Bildern von der Abbildungsvorrichtung (16) empfängt und die Schritte durchführt, die aufweisen:
Klassifizieren zumindest eines Bildes (106) aus der Vielzahl von Bildern basierend auf einer Kopfhaltung von zumindest einem Teil des Kopfes der Person (18) in Bezug auf zumindest ein Bild der Vielzahl von Bildern;
Überwachen hinsichtlich einer Bewegung von zumindest einem Auge (112) der Person, wenn das zumindest eine Bild als eine vorgegebene Klassifikation klassifiziert wird, wobei das Überwachen ein Charakterisieren einer Ausgenposition als eine X-Y-Koordinate auf einer kartesischen Koordinaten-Ebene umfasst; und
Bestimmen eines Aufmerksamkeitszustands (114) der Person (18) basierend auf der überwachten Bewegung von zumindest dem einen Auge, wobei die Bewegung des zumindest einen Auges über zumindest zwei Bilder der Vielzahl von Bildern überwacht wird, die von der Abbildungsvorrichtung (16) erlangt werden, wobei die Bewegung durch einen Bewegungsvektor auf der kartesischen Koordinaten-Ebene angezeigt wird;
wobei die Klassifikation des Bildes eines aus frontal und nicht-frontal aufweist; und
wobei die vorgegebene Klassifikation die frontale Klassifikation ist derart, dass zumindest eines der Augen überwacht wird, nachdem die Person (18) in zumindest einem Bild als die frontale Klassifikation klassifiziert wird.

2. Das Aufmerksamkeit-Erfassungssystem (10) gemäß Anspruch 1, wobei die Kopfhaltungs-Klassifikation aufweist ein Extrahieren zumindest eines Gesichtsmerkmals der Person (18) aus dem Bild und Vergleichen des zumindest einen extrahierten Gesichtsmerkmals mit einem Kopfhaltungsmodell.

3. Das Aufmerksamkeit-Erfassungssystem (10) gemäß Anspruch 1, wobei die Kopfhaltungs-Klassifikation aufweist ein Erfassen von zumindest einem Teil eines Gesichts der Person (18) und Klassifizieren des erfassten Gesichts durch eine vorgegebene Klassifikationsregel.

4. Das Aufmerksamkeit-Erfassungssystem (10) gemäß Anspruch 1, wobei der bestimmte Aufmerksamkeitszustand einer ist aus abgelenkt und nicht-abgelenkt.

5. Das Aufmerksamkeit-Erfassungssystem (10) gemäß Anspruch 1, wobei das Überwachen hinsichtlich einer Bewegung von zumindest einem Auge aufweist ein Vergleichen einer Länge des Bewegungsvektors mit einem ersten Schwellwert (156).

6. Das Aufmerksamkeit-Erfassungssystem (10) gemäß Anspruch 5, wobei das Überwachen hinsichtlich einer Bewegung von zumindest einem Auge weiter aufweist ein Vergleichen eines Werts eines Zählers mit einem zweiten Schwellenwert (162).

7. Das Aufmerksamkeit-Erfassungssystem (10) gemäß Anspruch 1, wobei das Aufmerksamkeit-Erfassungssystem (10) mit einem Fahrzeug (12) verwendet wird, um die Person (18) in dem Fahrzeug (12) zu erfassen.

8. Ein Verfahren (100) zum Erfassen einer Aufmerksamkeit einer Person (18), wobei das Verfahren (100) die Schritte aufweist:
Erlangen einer Vielzahl von Bildern (104) aus zumindest einem Teil einer Person (18);
Klassifizieren zumindest eines Bilds (106) der Vielzahl von Bildern basierend auf einer Kopfhaltung von zumindest einem Teil des Kopfes der Person (18) in Bezug auf zumindest ein Bild aus der Vielzahl von Bildern, wobei die Klassifikation der zumindest einen Bilds eines aus frontal und nicht-frontal aufweist;
Überwachen hinsichtlich einer Bewegung von zumindest einem Auge (112) der Person (18), wenn das zumindest eine Bild als eine vorgegebene Klassifikation klassifiziert wird, wobei das Überwachen ein Charakterisieren einer Ausgenposition als eine X-Y-Koordinate auf einer kartesischen Koordinaten-Ebene umfasst; und
Bestimmen eines Aufmerksamkeitszustands (114) der Person (18) basierend auf der überwachten Bewegung von zumindest dem einen Auge derart, dass der Aufmerksamkeitszustand eines aus abgelenkt und nicht-abgelenkt aufweist, wobei die Bewegung des zumindest einen Auges über zumindest zwei Bilder der Vielzahl von Bildern überwacht wird, wobei die Bewegung durch einen Bewegungsvektor auf der kartesischen Koordinaten-Ebene angezeigt wird;
wobei die vorgegebene Klassifikation für den durchzuführenden Schritt des Überwachens hinsichtlich einer Bewegung die frontale Klassifikation ist derart, dass zumindest eines der Augen überwacht wird, nachdem die Person (18) in zumindest dem einen Bild als die frontale Klassifikation klassifiziert wird.

9. Das Verfahren (100) gemäß Anspruch 8, wobei die Kopfhaltungs-Klassifikation aufweist ein Extrahieren zumindest eines Gesichtsmerkmals der Person (18) aus dem Bild und Vergleichen des zumindest einen extrahierten Gesichtsmerkmals mit einem Kopfhaltungsmodell.

10. Das Verfahren (100) gemäß Anspruch 8, wobei die Kopfhaltungs-Klassifikation aufweist ein Erfassen von zumindest einem Teil eines Gesichts der Person (18) und Klassifizieren des erfassten Gesichts durch eine vorgegebene Klassifikationsregel.

11. Das Verfahren (100) gemäß Anspruch 8, wobei der Schritt des Überwachens hinsichtlich einer Bewegung von zumindest einem Auge aufweist ein Vergleichen einer Länge des Bewegungsvektors mit einem ersten Schwellwert (156) derart, dass bestimmt wird, dass die Person (18) in einem ersten Aufmerksamkeitszustand (152) ist, wenn der Zählerwert größer ist als der erste Schwellenwert.

12. Das Verfahren (100) gemäß Anspruch 11, wobei der Schritt des Überwachens hinsichtlich einer Bewegung von zumindest einem Auge weiter aufweist ein Vergleichen eines Werts eines Zählers mit einem zweiten Schwellenwert (162) derart, dass bestimmt wird, dass die Person (18) in dem ersten Aufmerksamkeitszustand (152) ist, wenn der Zählerwert geringer ist als der zweite Schwellenwert, und dass die Person (18) in einem zweiten Aufmerksamkeitszustand (164) ist, wenn der Zählerwert größer ist als der zweite Schwellenwert.

13. Das Verfahren (100) gemäß Anspruch 8, wobei das Verfahren (100) mit einem Fahrzeug (12) verwendet wird, um eine Aufmerksamkeit der Person (18) in dem Fahrzeug (12) zu erfassen.

## Revendications

1. Système de détection de vigilance (10) comprenant :
un dispositif d'imagerie (16) positionné pour obtenir une pluralité d'images d'au moins une portion de la tête d'un sujet (18) ; et
un processeur de vigilance (28) en communication avec ledit dispositif d'imagerie (16) dans lequel ledit processeur de vigilance (28) reçoit ladite pluralité d'images depuis ledit dispositif d'imagerie (16) et exécute les étapes comprenant :
la classification d'au moins une image (106) de ladite pluralité d'images en se basant sur une pose de tête d'au moins une portion de la tête du dit sujet (18) à l'égard d'au moins une image de ladite pluralité d'images ;
la surveillance du mouvement d'au moins un oeil (112) dudit sujet si ladite au moins une image est classifiée selon une classification prédéterminée, dans laquelle ladite surveillance inclut une caractérisation d'un emplacement d'un oeil sous forme de coordonnées X-Y sur un plan de coordonnées cartésiennes ; et
la détermination d'un état de vigilance (114) dudit sujet (18) en se basant sur ledit mouvement surveillé dudit au moins un oeil, dans laquelle ledit mouvement dudit au moins un oeil est surveillé sur au moins deux images de ladite pluralité d'images obtenues par ledit dispositif d'imagerie (16), de sorte que ledit mouvement est indiqué par un vecteur de mouvement sur le plan de coordonnées cartésiennes ;
dans lequel ladite classification de ladite image comprend une classification parmi le type frontal et le type non-frontal ; et
dans lequel ladite classification prédéterminée est ladite classification de type frontal, de telle façon que ledit au moins un oeil est surveillé après que ledit sujet (18) ait été classifié dans ladite au moins une image comme une classification de type frontal.

2. Système de détection de vigilance (10) selon la revendication 1, dans lequel ladite classification de la pose de tête comprend l'extraction d'au moins une caractéristique faciale dudit sujet (18) depuis ladite image, et la comparaison de ladite au moins une caractéristique faciale extraite avec un modèle de pose de tête.

3. Système de détection de vigilance (10) selon la revendication 1, dans lequel ladite classification de pose de tête comprend la détection d'au moins une portion d'une face dudit sujet (18), et la classification de ladite face détectée par une règle de classification prédéterminée.

4. Système de détection de vigilance (10) selon la revendication 1, dans lequel ledit état de vigilance déterminé est un état parmi un état distrait et un état non distrait.

5. Système de détection de vigilance (10) selon la revendication 1, dans lequel ladite surveillance du mouvement d'au moins un oeil comprend la comparaison d'une longueur du vecteur de mouvement à une première valeur seuil (156).

6. Système de détection de vigilance (10) selon la revendication 5, dans lequel ladite surveillance du mouvement d'au moins un oeil comprend en outre la comparaison d'une valeur d'un compteur à une seconde valeur seuil (162).

7. Système de détection de vigilance (10) selon la revendication 1, dans lequel ledit système de détection de vigilance (10) est utilisé avec un véhicule (12) pour détecter ledit sujet (18) dans ledit véhicule (12)

8. Procédé (100) pour détecter la vigilance d'un sujet (18), ledit procédé (100) comprenant les étapes consistant à :
obtenir une pluralité d'images (104) d'au moins une portion d'un sujet (18) ;
classifier au moins une image (106) de ladite pluralité d'images en se basant sur une pose de tête d'au moins une portion de la tête dudit sujet (18) à l'égard d'au moins une image de ladite pluralité d'images, dans lequel ladite classification de ladite au moins une image comprend une classification parmi le type frontal et le type non frontal ;
surveiller le mouvement d'au moins un oeil (112) dudit sujet (18) si ladite au moins une image est classifiée selon une classification prédéterminée, dans lequel ladite surveillance inclut de caractériser un emplacement d'un oeil sous forme de coordonnées X-Y sur un plan de coordonnées cartésiennes ; et
déterminer un état de vigilance (114) dudit sujet (18) en se basant sur ledit mouvement surveillé dudit au moins un oeil, de telle façon que ledit état de vigilance comprend un état parmi un état distrait et un état non distrait, dans lequel ledit mouvement dudit au moins un oeil est surveillé sur au moins deux images de ladite pluralité d'images, et ledit mouvement est indiqué par un vecteur de mouvement sur le plan de coordonnées cartésiennes ;
dans lequel ladite classification prédéterminée pour ladite étape de surveillance de mouvement à exécuter est ladite classification frontale, de telle façon qu'au moins un des yeux est surveillé après que ledit sujet (18) dans ladite au moins une image ait été classifié selon ladite classification frontale.

9. Procédé (100) selon la revendication 8, dans lequel ladite classification de la pose de tête comprend l'extraction d'au moins une caractéristique faciale dudit suj et (18) depuis ladite image, et la comparaison de ladite au moins une caractéristique faciale extraite à un modèle de pose de tête.

10. Procédé (100) selon la revendication 8, dans lequel ladite classification de pose de tête comprend la détection d'au moins une portion d'une face dudit sujet (18), et la classification de ladite face détectée par une règle de classification prédéterminée.

11. Procédé (100) selon la revendication 8, dans lequel ladite étape consistant à surveiller le mouvement d'au moins un oeil comprend la comparaison d'une longueur du vecteur de mouvement à une première valeur seuil (156), de sorte que l'on détermine que ledit sujet (18) est dans un premier état de vigilance (152) quand ladite valeur de compteur est supérieure à ladite première valeur seuil.

12. Procédé (100) selon la revendication 11, dans lequel ladite étape consistant à surveiller le mouvement d'au moins un oeil comprend encore la comparaison d'une valeur d'un compteur à une seconde valeur seuil (162), de telle façon que l'on détermine que ledit sujet (18) est dans ledit premier état de vigilance (152) quand ladite valeur de compteur est inférieure à ladite seconde valeur seuil, et que ledit sujet (18) est dans un second état de vigilance (164) quand ladite valeur de compteur est supérieure à ladite seconde valeur seuil.

13. Procédé (100) selon la revendication 8, dans lequel ledit procédé (100) est utilisé avec un véhicule (12) pour détecter une vigilance dudit sujet (18) dans ledit véhicule (12)
